Europäisches Patentamt

European Patent Office

Office européen des brevets

(19)

(11) Publication number: **0 019 638**
**B1**

(12) **EUROPEAN PATENT SPECIFICATION**

(45) Date of publication of patent specification: **02.01.85**

(21) Application number: **79901094.7**

(22) Date of filing: **04.09.79**

(88) International application number:
**PCT/JP79/00234**

(87) International publication number:
**WO 80/00574 03.04.80 Gazette 80/07**

(51) Int. Cl.⁴: **C 12 Q 1/00, C 12 N 11/00,**
**G 01 N 33/50, G 01 N 35/00**

(54) **IMMOBILIZED ENZYME COLUMN.**

(30) Priority: **06.09.78 JP 110102/78**
**08.06.79 JP 78767/79**

(43) Date of publication of application:
**10.12.80 Bulletin 80/25**

(45) Publication of the grant of the patent:
**02.01.85 Bulletin 85/01**

(84) Designated Contracting States:
**CH DE FR GB NL SE**

(56) References cited:
**CH-A- 453 568**
**JP-A-49 057 893**
**JP-A-52 043 487**
**JP-A-52 062 088**
**JP-B-44 000 592**
**US-A-3 743 103**

The file contains technical information
submitted after the application was filed and
not included in this specification

(73) Proprietor: **Fujisawa Pharmaceutical Co., Ltd.**
**3, Doshomachi 4-chome Higashi-ku**
**Osaka-shi, Osaka 541 (JP)**

(72) Inventor: **NAKAJIMA, Takao**
**4-22, Saidaiji-shinmachi 2-chome**
**Nara-shi, Nara 631 (JP)**
Inventor: **TERADA, Masafumi**
**3-10, Hishiya-higashi 1-chome**
**Higashiosaka-shi Osaka 577 (JP)**
Inventor: **MORI, Tohru**
**1-8-206, Midorigaoka 2-chome**
**Ikeda-shi Osaka 563 (JP)**

(74) Representative: **Patentanwälte TER MEER -**
**MÜLLER - STEINMEISTER**
**Triftstrasse 4**
**D-8000 München 22 (DE)**

(56) References cited:
**Chem. Abstracts, vol. 88, 1978, page 218,**
**abstract 132895x, Columbus, Ohio, US, P.V.**
**SUNDARAM et al.: "Immobilized enzyme**
**nyion-tube reactors for routine determination**
**of urea and citrulline in serum**

Courier Press, Leamington Spa, England.

## Description

This invention relates to an immobilized enzyme column useful for automatic chemical analyzers by which substances contained in the blood serum, urine or the like are analyzed with use of an enzyme for clinical tests.

Background art

An immobilized enzyme column has a sample supply tube connected to its rear end and a sample discharge tube connected to its front end. Each of the tubes is divided, at a position close to the column, into two segments which are connected together by a three-way joint at two socket portions thereof. An air bubble removing tube is joined to the remaining one socket portion of the joint on the sample supply tube, and an air bubble supplying tube is joined to the remaining one socket portion of the joint on the sample discharge tube. Connected to a rear portion of the sample supply tube is an air supply tube for intermittently supplying air to divide a sample with air bubbles. When different samples are supplied to the column, a preceding sample will mingle with the following different sample, giving an inaccurate value on analysis. To avoid this, the sample is divided by air bubbles. In practice, however, it is difficult to interpose an air bubble accurately between the preceding sample and the following sample. Further if the sample passes through an immobilized enzyme with an air bubble incorporated in the sample, the bubble will diffuse through the sample, adversely affecting the analyzed value, so that there is the need to remove the bubble from the sample before the inlet to the portion of the column where the enzyme is accommodated and to supply an air bubble again to the sample after it has passed through the enzyme. Removal of the bubble nevertheless permits the preceding sample to mingle with the following sample and makes it meaningless to introduce the bubble into the sample. To eliminate such a problem, air is supplied to the same sample so that some dividing air bubbles will be present therein at specified spacing. While the immobilized enzyme accommodating portion in the column is spaced from the bubble removing tube and from the bubble supplying tube, the shorter the spacings, the better, because the longer the spacings, the longer will be the sections in which the preceding sample becomes mixed with the following one and the lesser will be the sample portion to be analyzed reliably. To compensate for this, there is the need to use an increased amount of sample which takes a longer period of time for analysis.

Disclosure of invention

The immobilized enzyme columns according to this invention include at least one column main body, an air bubble inlet disposed close to a sample outlet to communicate with a channel extending from a sample inlet to the sample outlet, an air bubble outlet disposed close to the sample inlet to communicate with the channel, and an immobilized enzyme accommodating portion formed in the channel between the bubble inlet and the bubble outlet and partitioned by a filter at each end thereof. This arrangement minimizes the spacing between the immobilized enzyme accommodating portion in the column and an air bubble removing tube, and the spacing between the accommodating portion and an air bubble supplying tube, permitting analysis of a smaller quantity of sample with higher accuracy in a shorter period of time than heretofore possible.

The immobilized enzyme columns of this invention include one comprising a plurality of column main bodies which are arranged side by side. The column main bodies adjacent to each other are formed on their opposed sides with open connecting portions which are connected together. This arrangement is convenient for using a plurality of kinds of enzymes at the same time without the necessity of interconnecting columns with a tube as heretofore done and further assures more accurate analysis than when enzymes are used in the form of a mixture. The arrangement is economical because when one of the enzymes has been inactivated earlier than the other, the column with the inactivated enzyme alone can be replaced.

Brief description of the drawings

·Fig. 1 is a diagram schematically showing an automatic chemical analyzer in its entirety;

Fig. 2 is a plan view of an immobilized enzyme column of this invention comprising one column main body;

Fig. 3 is a view in section taken along the line III—III in Fig. 2;

Figs. 4 and 5 are sectional views corresponding to Fig. 3 and showing modified immobilized enzyme columns each comprising one column main body;

Fig. 6 is a sectional view corresponding to Fig. 3 and showing an immobilized enzyme column of this invention comprising two column main bodies;

Fig. 7 is a side elevation partly broken away and showing a modification of the same column; and

Fig. 8 is a graph showing the influence of a preceding sample on the following sample during analysis.

Best mode of carrying out the invention

This invention will be described below in greater detail with reference to the accompanying drawings.

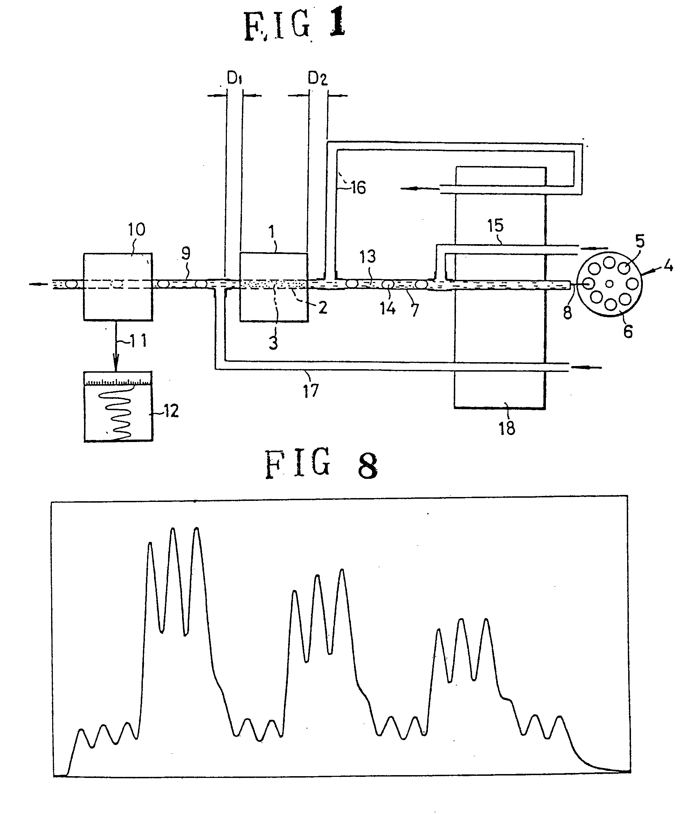

Fig. 1 shows an automatic chemical analyzer in its entirety. An immobilized enzyme column 1 of the conventional type has an immobilized enzyme column accommodating portion 2 in the form of a horizontal channel extending through the center of the column. An enzyme 3 immobilized on a carrier, such as porous glass, is packed in the portion 2. A sampler 4 comprises a circular table 6 intermittently rotatable and having a large number of sample cups 5 in its marginal portion. Connected to the rear end of the column 1 is the front end of a sample supply tube 7 having a rear end communicating with a sample cup 5 via a withdrawing member 8. Connected to the front end of the column 1 is the rear end of a sample discharge tube 9. A colorimeter 10 is provided between the front end outlet thereof and the rear end. The measurement obtained by the colorimeter 10 is sent as an electric signal 11 to a recorder 12. An air supply tube 15 for intermittently supplying air to divide a sample 13 with air bubbles 14 is connected to the supply tube 7 at a portion thereof close to its rear end. Each of the tubes 7, 9 is divided, at a position close to the column 1, into two segments which are connected together by a three-way joint at two socket portions thereof. An air bubble removing tube 16 is joined to the remaining one socket portion of the joint on the sample supply tube 7, and an air bubble supplying tube 17 is joined to the remaining one socket portion of the joint on the sample discharge tube 9. A metering pump 18 is associated with the tubes 7, 15, 16 and 17. The accommodating portion 2 is spaced from the bubble removing tube 16 by a distance D2 and from the bubble supplying tube 17 by a distance D1.

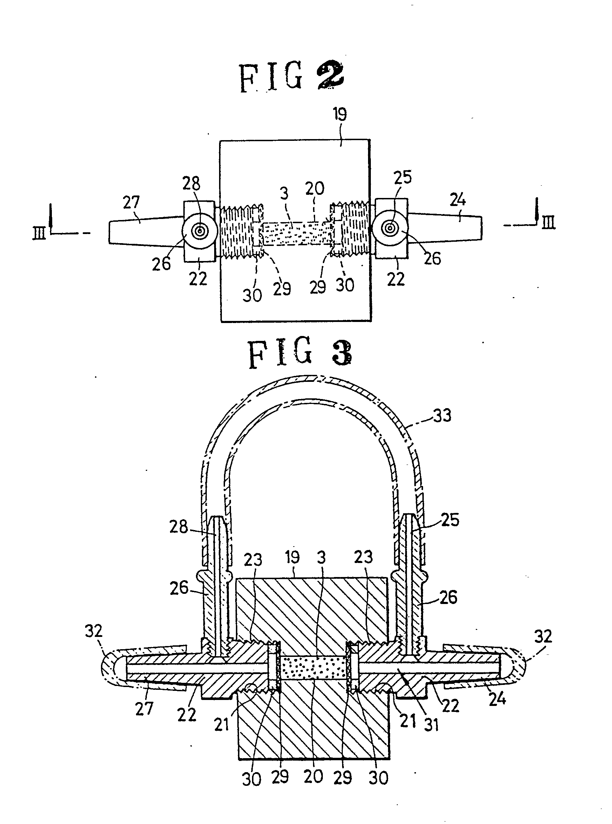

Figs. 2 and 3 show an immobilized enzyme column of this invention comprising one column main body. The main body 19 is rectangular parallelepipedal and is formed in its center with an immobilized enzyme accommodating portion 20 in the form of a horizontal hollow space. Formed in front and rear portions of the column main body 19 are internally threaded portions 21 communicating with the accommodating portion 20 and having a larger diameter than the portion 20. A tubular member 22 has at its base end an externally threaded portion 23 which is screwed into each of the internally threaded portions 21. The tubular member 22 on the rear side of the main body 19 has a forward end serving as a sample inlet 24. A nozzle 26 having an air bubble outlet 25 at its forward end is screwed into the same tubular member at a position close to the base end thereof. The tubular member 22 on the front side of the main body has a forward end serving as a sample outlet 27 and also has screwed in a portion thereof close to its base end a nozzle 26 having an air bubble inlet 28 at its forward end. Provided in the innermost part of each internally threaded portion 21 are a filter 29 and an annular packing 30 as arranged side by side. With the tubular members 22 screwed in the front and rear portions of the main body 19, the column has a channel 31 extending from the sample inlet 24 to the sample outlet 27. The accommodating portion 20 is formed in the channel 31, as partitioned by the filters 29 at its opposite ends. The portion 20 may have the immobilized enzyme 3 accommodated therein in advance, or may accommodate the enzyme 3 when the column is to be used. The column main body 19, tubular members 22 and nozzles 26 are all made of transparent rigid synthetic resin. For the identification of the enzyme in the column main body 19, the tubular members 22 and nozzles 26 are preferably colored in accordance with the kind of the enzyme. To prevent the inactivation of the enzyme 3 before the column is set on an analyzer, the sample inlet 24 and outlet 27 are each closed with a soft synthetic resin cap 32, and an inverted U-shaped bent tube 33 of soft synthetic resin is fitted at its ends to the bubble inlet 28 and outlet 25, as illustrated in broken lines in Fig. 3.

To incorporate the column of this invention into the analyzer, the caps 32 and tube 33 are removed, and the sample supply tube 7 is fitted to the sample inlet 24, the sample discharge tube 9 to the sample outlet 27, the air bubble supplying tube 17 to the bubble inlet 28, and the air bubble removing the tube 16 to the bubble outlet 25.

When different samples are supplied to the column, a preceding sample will affect the following sample. Now aqueous solutions of glucose having varying concentrations are used as samples for the measurement of the concentrations, as shown in the table below. Fig. 8 shows the results, in which the concentration is plotted as ordinate vs. time as abscissa. The concentration curve shown is obtained by using a conventional immobilized enzyme column, supplying a wash liquor (water) and the sample alternately and also supplying three samples for each concentration. When samples of low concentration are followed by samples of high concentration, the first sample of high concentration is affected by the preceding sample of low concentration and exhibits a lower concentration than is specified. Conversely when samples of high concentration are followed by samples of low concentration, the first sample of low concentration is affected by the preceding sample of high concentration, exhibiting a higher concentration than is specified. After a sample of high concentration has been supplied, the wash liquor is of course fed to the column before a sample of low concentration is supplied, but the resulting wash liquor has a higher concentration than the sample of low concentration. The following table shows the influence of preceding samples on the following samples as determined with use of the conventional immobilized enzyme column and the column of this invention for comparison. The sample supplied the third time, which has the specified concentration, is used as the standard to determine the influence of the preceding sample on the first sample, namely the degree of decrease or increase in the concentration. The immobilized enzyme accommodating portion is spaced from both the bubble removing tube and the bubble supplying tube by 10 cm in the case of the conventional column, and by 5 mm with the column of this invention shown in Figs. 2 and 3.

| | Degree of influence (%) | |
|---|---|---|
| Change of sample | Product of the invention | Conventional product |
| 100 mg/dl→500 mg/dl | —0.8 | —5.1 |
| 500 mg/dl→100 mg/dl | 17.4 | 55.0 |
| 100 mg/dl→400 mg/dl | —2.7 | —8.4 |
| 400 mg/dl→100 mg/dl | 16.5 | 44.0 |
| 100 mg/dl→300 mg/dl | —4.6 | —5.9 |
| 300 mg/dl→100 mg/dl | 7.1 | 26.5 |

The above table reveals that the degrees of influence of the preceding samples on the following samples are much smaller with the column of the invention than with the conventional column.

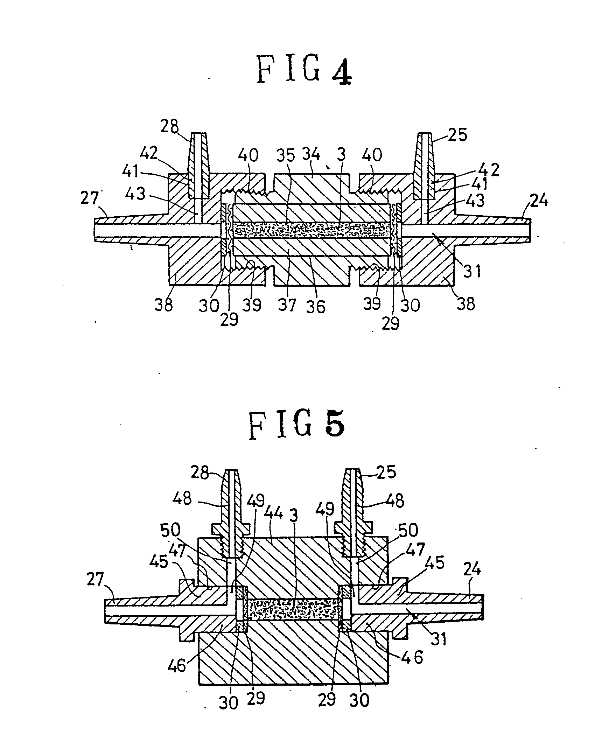

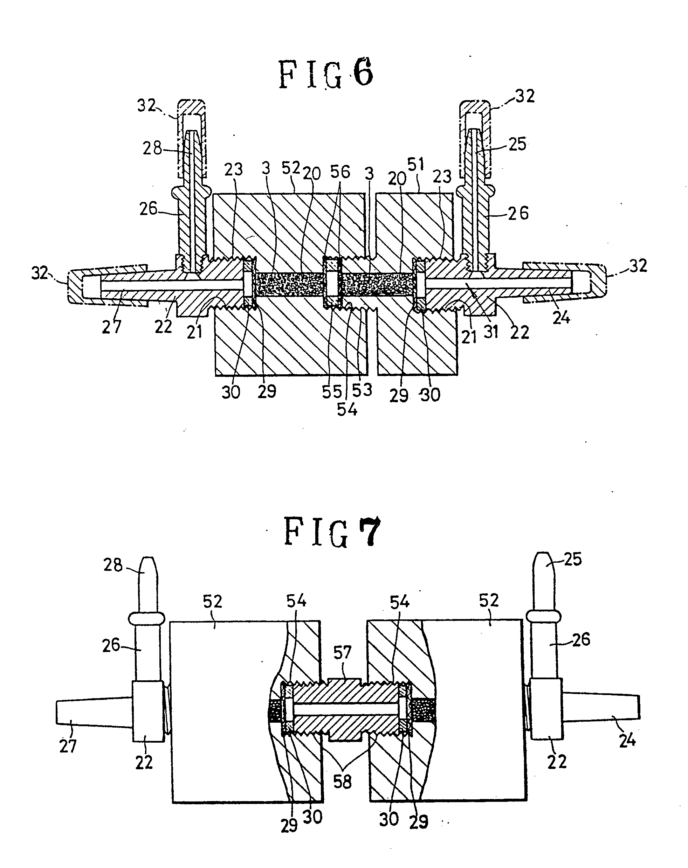

Figs. 4 and 5 show modified immobilized enzyme columns of this invention each comprising one column main body. With the column shown in Fig. 4, a tube 37 is withdrawably inserted in a bore 36 extending through a column main body 34 to provide therein an immobilized enzyme accommodating portion 35. Two tubular members 38 have the same outside diameter as the column main body 34 except for a sample inlet 24 and a sample outlet 27 and each have an externally threaded portion 39. The internally threaded portions 39 are screwed on externally threaded portions 40 at the front and rear ends of the main body 34. A nozzle 42 has a base end intimately fitted in a cavity 41 formed in a top portion of each of the tubular members 38. Preferably the base end is bonded to the bottom of the cavity 41 with adhesive. The tubular members 38 are formed with passages 43 for causing an air bubble outlet 25 and an air bubble inlet 28 to communicate with a channel 31. If a filter 29 and a packing 30 are adhered to one end of the tube 37, it is convenient to place an immobilized enzyme 3 into the accommodating portion 35 of the tube 37 after withdrawing the tube 37 from the column main body 34. The main body 34 may be merely formed with a bore extending therethrough and serving as part of the channel 31 to thereby provide an accommodating portion for the immobilized enzyme. Throughout the drawings, like parts or members are referred to by like reference numerals.

The column shown in Fig. 5 has a column main body 44 which itself is similar in shape to the one shown in Figs. 2 and 3. Each tubular member 45 has at its base end a straight tubular fitting portion 46 which is intimately fittable in a circular cavity 47 formed in the column main body 44. To place an immobilized enzyme 3 into an accommodating portion 20, the fitting portion 46 can be withdrawn from the cavity 47. Nozzles 48 having an air bubble outlet 25 and an air bubble inlet 28 are screwed in the top of the main body 44 to extend upward therefrom. Grooves 49 are formed in the base ends of the tubular members 45 to extend toward the nozzles 48. The main body 44 is formed with passages 50 for holding the grooves 49 in communication with the nozzles 48.

Fig. 6 shows an immobilized enzyme column comprising two column main bodies according to this invention. The two main bodies 51, 52 of Fig. 6 themselves are similar in shape to the main body 19 shown in Figs. 2 and 3 and are arranged with their channels in alignment. The main bodies 51, 52 adjacent to each other are formed on their opposed sides with open connecting portions 53, 54. The connecting portion 53 is provided with an externally threaded portion, while the other connecting portion has an internally threaded portion for the externally threaded portion to be screwed in. A packing 55 is sandwiched between filters 56, and the resulting assembly is interposed between these threaded portions screwed together, whereby the two column main bodies 51, 52 are joined together.

Fig. 7 shows a modification of the column of Fig. 6. Each of two column main bodies 52 has an internally threaded, open connecting portion 54. A tubular connecting member 57 has at its opposite ends externally threaded portions 58, which are screwed in the internally threaded portions, whereby the two column main bodies are connected together.

While Figs. 6 and 7 each show two column main bodies which are connected together, at least three column main bodies can be connected together with use of an intermediate column main body having no tubular member.

Industrial applicability

The immobilized enzyme columns of this invention are suited for use in automatic chemical analyzers for analyzing substances contained in the blood serum, urine or the like with use of an enzyme for clinical tests.

**Claims**

1. An immobilized enzyme column useful for automatic chemical analyzers comprising a sample supply tube connected to its rear end, a sample discharge tube connected to its front end, an air bubble removing tube joined to the sample supply tube and an air supplying tube joined to the sample discharge tube, characterized in that the column includes at least one column main body (19, 34 or 44, 51 and 52) wherein the air bubble inlet (28) is disposed close to the sample outlet (27) to communicate with a channel (31) extending from the sample inlet (24) to the sample outlet (27), and the air bubble outlet (25) is disposed close to the sample inlet (24) to communicate with the channel (31), and wherein an immobilized enzyme accommodating portion (20 or 35) is formed in the channel between the bubble inlet (28) and the bubble outlet (25) and partitioned by a filter (29) at each end thereof.

2. An immobilized enzyme column as defined in claim 1 characterized in that the enzyme accommodating portion (35) is provided within a tube (37) withdrawably fitted in a bore (36) extending through the column main body (34).

3. An immobilized enzyme column as defined in claim 1 characterized in that the bubble inlet (28) and the bubble outlet (25) are provided on the column main body (44).

4. An immobilized enzyme column as defined in claim 1 characterized in that the sample inlet (24) is provided on one tubular member (22, 38 or 45) and that the sample outlet (27) is provided on another tubular member (22, 38 or 45), each tubular member (22, 38 or 45) being detachably mounted on the column main body (19, 34, 44, 51 or 52) so as to be connected to the enzyme accommodating portion (20 or 35).

5. An immobilized enzyme column as defined in claim 4 characterized in that the tubular member (22) is provided at its base end with an externally threaded portion (23) and that the column main body (19) is formed with an internally threaded portion (21) for the externally threaded portion (23) to be screwed in.

6. An immobilized enzyme column as defined in claim 4 characterized in that the tubular member (38) is formed in its base end with an internally threaded portion (39) and that the column main body (34) is provided with an externally threaded portion (40) for the internally threaded portion (39) to be screwed thereon.

7. An immobilized enzyme column as defined in claim 4 characterized in that the tubular member (45) is provided at its base end with a fitting portion (46) and that the column main body (44) is formed with a cavity (47) for the fitting portion (46) to fit in intimately.

8. An immobilized enzyme column as defined in claim 4 characterized in that the tubular member (38) having the sample inlet (24) is provided with the bubble outlet (25) and that the tubular member (38) having the sample outlet (27) is provided with the bubble inlet (28).

9. An immobilized enzyme column as defined in claim 4 characterized in that a packing (30) in contact with the filter (29) is provided between the tubular member (22, 38 or 45) and the column main body (19, 34, 44, 51 or 52).

10. An immobilized enzyme column as defined in any one of claims 1 to 9 characterized in that a plurality of column main bodies (51 and 52) are arranged side by side and that the column main bodies (51 and 52) adjacent to each other are each formed on their opposed sides with an open connecting portion (53 or 54), the open connecting portions (53 and 54) being connected together.

11. An immobilized enzyme column as defined in claim 10 characterized in that one connecting portion (53) is provided with an externally threaded portion and that the other connecting portion (54) is provided with an internally threaded portion for the externally threaded portion to be screwed in.

12. An immobilized enzyme column as defined in claim 10 characterized in that both open connecting portions (54) are each provided with an internally threaded portion and that externally threaded portions (58) provided on both ends of a tubular connecting member (57) are screwed in the internally threaded portions.

**Patentansprüche**

1. Säule mit immobilisiertem Enzym für automatische chemische Analysengeräte, mit einem an ihrem hinteren Ende angeordneten Probenzuführungsrohr, einem an ihrem vorderen Ende angeordneten Probenabführungsrohr, einem mit dem Probenzuführungsrohr verbundenen Luftblasenabführungsrohr und einem mit dem Probenabführungsrohr verbundenen Luftzuführungsrohr, dadurch gekennzeichnet, daß die Säule mindestens einen Säulenhauptkörper (19, 34 oder 44, 51 und 52) aufweist, in dem der Luftblaseneinlaß (28) in der Nähe des Probenauslasses (27) angeordnet ist und mit einem Kanal (31) in Verbindung steht, der sich von dem Probeneinlaß (24) zum Probenauslaß (27) erstreckt, und der Luftblasenauslaß (25) in der Nähe des Probeneinlasses (24) angeordnet ist und mit dem Kanal (31) in Verbindung steht, und in dem Kanal zwischen dem Blaseneinlaß (28) und dem Blasenauslaß (25) ein das immobilisierte Enzym aufnehmender Bereich (20 oder 35) vorgesehen ist, der an jedem Ende durch ein Filter (29) abgeteilt ist.

2. Säule mit immobilisiertem Enzym nach Anspruch 1, dadurch gekennzeichnet, daß der das Enzym aufnehmende Bereich (35) in einem Rohr (37) vorgesehen ist, welches herausziehbar in einer Bohrung (36) angeordnet ist, welche sich durch den Säulenhauptkörper (34) erstreckt.

3. Saule mit immobilisiertem Enzym nach Anspruch 1, dadurch gekennzeichnet, daß der Blaseneinlaß (28) und der Blasenauslaß (25) am Säulenhauptkörper (44) angeordnet sind.

4. Säule mit immobilisiertem Enzym nach Anspruch 1, dadurch gekennzeichnet, daß der Probeneinlaß (24) an einer röhrenförmigen Einrichtung (22, 38 oder 45) angeordnet ist und der Probenauslaß (27) an einer anderen röhrenförmigen Einrichtung (22, 38 oder 45) angeordnet ist, wobei die röhrenförmigen Einrichtungen (22, 38 oder 45) derart lösbar an dem Säulenhauptkörper (19, 34, 44, 51 oder 52) befestigt sind, daß sie mit dem das Enzym aufnehmenden Bereich (20 oder 35) verbunden sind.

5. Säule mit immobilisiertem Enzym nach Anspruch 4, dadurch gekennzeichnet, daß die röhrenförmige Einrichtung (22) an ihrem Verbindungsende mit einem Außengewindebereich (23) versehen ist und der Säulenhauptkörper (19) mit einem Innengewindebereich (21) zum Einschrauben des Außengewindebereiches (23) versehen ist.

6. Säule mit immobilisiertem Enzym nach Anspruch 4, dadurch gekennzeichnet, daß die röhrenförmige Einrichtung (38) an ihrem Verbindungsende mit einem Innengewindebereich (39) versehen ist und der Säulenhauptkörper (34) mit einem Außengewindebereich (40) zum Einschrauben des Innengewindebereiches (39) versehen ist.

7. Säule mit immobilisiertem Enzym nach Anspruch 4, dadurch gekennzeichnet, daß die röhrenförmige Einrichtung (45) an ihrem Verbindungsende mit einem Verbindungsbereich (46) versehen ist und der Säulenhauptkörper (44) einen Hohlraum (47) zur passenden Aufnahme des Verbindungsbereiches (46) aufweist.

8. Säule mit immobilisiertem Enzym nach Anspruch 4, dadurch gekennzeichnet, daß die röhrenförmige Einrichtung (38) mit dem Probeneinlaß (24) mit dem Blasenauslaß (25) versehen ist und die röhrenförmige Einrichtung (38) mit dem Probenauslaß (27) mit dem Blaseneinlaß (28) versehen ist.

9. Säule mit immobilisiertem Enzym nach Anspruch 4, dadurch gekennzeichnet, daß zwischen der röhrenförmigen Einrichtung (22, 38 oder 45) und dem Säulenhauptkörper (19, 34, 44, 51 oder 52) eine mit dem Filter (29) in Kontakt stehende Packung vorgesehen ist.

10. Säule mit immobilisiertem Enzym nach einem der Ansprüche 1 bis 9, dadurch gekennzeichnet, daß eine Vielzahl von Säulenhauptkörpern (51 und 52) nebeneindander angeordnet sind und daß benachbarte Säulenhauptkörper (51 und 52) nebeneinander angeordnet sind und daß benachbarte Säulehauptkörper (51, und 52) an ihren gegenüberliegenden Seiten mit einem offenen Verbindungsbereich (53 oder 54) versehen sind, welche offenen Verbindungsbereiche (53 und 54) miteinander verbunden sind.

11. Säule mit immobilisiertem Enzym nach Anspruch 10, dadurch gekennzeichnet, daß ein Verbindungsbereich (53) mit einem Außengewindebereich und der andere Verbindungsbereich (54) mit einem Innengewindebereich zum Einschrauben des Außengewindebereiches versehen sind.

12. Säule mit immobilisiertem Enzym nach Anspruch 10, dadurch gekennzeichnet, daß die beiden offenen Verbindungsbereiche (54) jeweils mit einem Innengewindebereich versehen sind und an beiden Enden der röhrenförmigen Verbindungseinrichtung (57) Außengewindebereiche (58) zum Einschrauben in die Innengewindebereiche vorgesehen sind.

## Revendications

1. Colonne à enzyme immobilisée utile pour des analyseurs chimiques automatiques comprenant un tube d'amenée d'échantillons relié à son extrémité arrière, un tube d'évacuation d'échantillons relié à son extrémité avant, un tube d'élimination de bulles d'air relié au tube d'amenée d'échantillons et un tube d'amenée d'air relié au tube d'évacuation d'échantillons, caractérisée en ce qu'elle comporte au moins un corps principal de colonne (19, 34 ou 44, 51 et 52) dans lequel l'entrée de bulles d'air (28) est disposée près de la sortie d'échantillons (27) pour communiquer avec un canal (31) s'étendant de l'entrée d'échantillons (24) à la sortie d'échantillons (27), et la sortie de bulles d'air (25) est disposée près de l'entrée d'échantillons (24) pour communiquer avec le canal (31), et dans lequel un tronçon de réception d'enzyme immobilisée (20 ou 35) est formé dans le canal entre l'entrée de bulles (28) et la sortie de bulles (25) et cloisonné par un filtre (29) à chacune de ses extrémités.

2. Colonne à enzyme immobilisée selon la revendication 1, caractérisée en ce que le tronçon de réception d'enzyme (35) est ménagé dans un tube (37) inséré de manière amovible dans un alésage (36) traversant le corps principal de colonne (34).

3. Colonne à enzyme immobilisée selon la revendication 1, caractérisée en ce que l'entrée de bulles (28) et la sortie de bulles (25) sont prévues sur le corps principal de colonne (44).

4. Colonne à enzyme immobilisée selon la revendication 1, caractérisée en ce que l'entrée d'échantillons (24) est prévue sur un élément tubulaire (22, 38 ou 45) et la sortie d'échantillons (27) est prévue sur un autre élément tubulaire (22, 38 ou 45), chaque élément tubu-

**0 019 638**

laire (22, 38 ou 45) étant monté de manière amovible sur le corps principal de colonne (19, 34, 44, 51 ou 52) de façon à être relié au tronçon de réception d'enzyme (20 ou 35).

5. Colonne à enzyme immobilisée selon la revendication 4, caractérisée en ce que l'élément tubulaire (22) présente à son extrémité de base un tronçon fileté (23) et en ce que le corps principal de colonne (19) présente un taraudage (21) pour le vissage dans son intérieur de tronçon fileté (23).

6. Colonne à enzyme immobilisée selon la revendication 4, caractérisée en ce que l'élément tubulaire (38) présente dans son extrémité de base un tronçon taraudé (39) et en ce que le corps principal de colonne (34) présente un filetage (40) pour le vissage sur son extérieur de tronçon taraudé (39).

7. Colonne à enzyme immobilisée selon la revendication 4, caractérisée en ce que l'élément tubulaire (45) présente à son extrémité de base un tronçon d'emboîtement (46) et en ce que le corps principal de colonne (44) présente une cavité (47) pour l'emboîtement intime du tronçon d'emboîtement (46) dans son intérieur.

8. Colonne à enzyme immobilisée selon la revendication 4, caractérisée en ce que l'élément tubulaire (38) présentant l'entrée d'échantillons (24) est muni de la sortie de bulles (25) et en ce que l'élément tubulaire (38) présentant la sortie d'échantillons (27) est muni de l'entrée de bulles (28).

9. Colonne à enzyme immobilisée selon la revendication 4, caractérisée en ce qu'une bague de garniture (30) en contact avec le filtre (29) est prévue entre l'élément tubulaire (22, 38 ou 45) et le corps principal de colonne (19, 34, 44, 51 ou 52).

10. Colonne à enzyme immobilisée selon l'une quelconque des revendications 1 à 9, caractérisée en ce qu'une pluralité de corps principaux de colonne (51 et 52) sont disposés côte à côte et en ce que les corps principaux de colonne (51 et 52) situés l'une près de l'autre présentent chacun sur leurs côtés en regard un tronçon de raccordement ouvert (53 ou 54), les tronçons de raccordement ouverts (53 et 54) étant reliés l'une à l'autre.

11. Colonne à enzyme immobilisée selon la revendication 10, caractérisée en ce qu'un tronçon de raccordement (53) présente un tronçon fileté et l'autre tronçon de raccordement (54) présente un tronçon taraudé pour le vissage dans son intérieur du tronçon fileté.

12. Colonne à enzyme immobilisée selon la revendication 10, caractérisée en ce que les deux tronçons de raccordement ouverts (54) présentent chacun un taraudage et en ce que des filetages (58) prévus sur les deux extrémités d'un élément de raccordement tubulaire (57) sont vissés dans les tronçons taraudés.

# F.IG 1

# FIG 8

# FIG 2

# FIG 3

## FIG 4

## FIG 5

# FIG 6

# FIG 7